# FASCICULE DE BREVET EUROPEEN

(11) **EP 1 182 180 B1**
(45) Date de publication et mention de la délivrance du brevet: **12.05.2010**
(21) Numéro de dépôt: 01402178.6
(22) Date de dépôt: 14.08.2001
(51) Int. Cl.: C07B 35/04, C07C 5/333, C07C 11/02, C07C 13/20

(54) **Procédé de deshydrogénation de composés organiques en présence d'un catalyseur bimétallique**
Verfahren zur Dehydrierung von organischen Verbindungen in Gegenwart eines bimetallischen Katalysators
Process for the dehydrogenation of organic compounds in the presence of a bimetallic catalyst

(30) Priorité: 23.08.2000 FR 0010879
(43) Date de publication de la demande: 27.02.2002
(73) Titulaire: Institut Français du Pétrole, 92852 Rueil-Malmaison Cedex (FR)
(72) Inventeur: Le Peltier, Fabienne, 92500 Rueil Malmaison (FR); Didillon, Blaise, 69340 Francheville (FR); Jumas, Jean-Claude, 34830 Jacou (FR); Olivier-Fourcade, Josette, 34830 Jacou (FR)

(56) Documents cités:
- EP-A- 0 248 130
- US-A- 3 531 543
- US-A- 3 691 102
- US-A- 4 716 143
- M. C. HOBSON: "A Mössbauer spectoscopy study of platinum tin reforming catalysts" JOURNAL OF CATALYSIS, vol. 142, 1993, pages 641-654, XP002158598 MN US

## Description

### Domaine technique

La présente invention concerne un procédé pour la déshydrogénation de composés organiques en particulier de paraffines et de naphtènes en vue de produire des alcènes et des composés aromatiques avec des rendements optimum. Le procédé de l'invention est réalisé en présence d'un catalyseur bimétallique supporté comportant un métal du groupe VIII et au moins un métal additionnel constitué par l'étain, au moins une partie de l'étain étant en forte interaction avec ledit métal du groupe VIII.

Les aromatiques et les alcènes constituent une charge de choix pour l'industrie pétrochimique.

Les procédés de déshydrogénation de paraffines légères permettent de valoriser des hydrocarbures aliphatiques à bas point d'ébullition tels que par exemple les butanes et isobutanes, les pentanes et isopentanes que l'on peut récupérer après extraction des insaturés des coupes de vapocraquage ou de craquage catalytique. Le procédé de déshydrogénation de paraffines plus longues est un procédé commercial important du fait de la demande actuelle en monooléfines pour la préparation de détergents biodégradables ou de produits pharmaceutiques par exemple.

Si les principales sources d'alcènes sont les procédés de craquage catalytique et de vapocraquage, ces deux procédés produisent également des sous-produits et la demande croissante étant orientée vers des alcènes spécifiques, il serait peu économique de les produire par craquage.

C'est pourquoi la production directe d'alcènes reste dans certains cas une étape incontournable. C'est le cas du propylène, isobutène ou des alcènes linéaires à chaîne longue pour la production polypropylène, de MTBE et de LAB (Linear Alkyl Benzene) respectivement.

Les principales spécificités de la réaction de déshydrogénation des paraffines résident dans le fait que l'équilibre thermodynamique limite le taux de conversion par passe et que la réaction est fortement endothermique. Ces deux caractéristiques sont déterminantes dans les choix technologiques au niveau du procédé mais également dans la composition, la structure et la conception du catalyseur.

Le fait de travailler à haute température est nécessaire pour maintenir un niveau de conversion proche de l'équilibre thermodynamique, mais ces hautes températures favorisent également un certain nombre de réactions parasites conduisant à un produit de moindre qualité. Parmi ces réactions, il existe des réactions conduisant à la formation de produits légers (craquage, hydrogénolyse), de composés fortement insaturés précurseurs de dépôts carbonés et donc initiateurs de désactivation (déshydrocyclisation, déshydrogénation profonde) comme les composés aromatiques ou les dioléfines et des réactions d'isomérisation squelettale responsables de la formation de molécules ramifiées. Dans ces conditions opératoires particulièrement sévères, il est très difficile de maintenir une activité élevée pendant des durées importantes en raison de ces réactions secondaires.

### Art antérieur

Les moyens de limiter ces réactions secondaires peuvent porter sur le procédé et / ou sur la formulation catalytique. Ainsi en vue d'améliorer les performances de systèmes catalytiques et notamment leur stabilité, le brevet US 4,716,143 décrit un catalyseur à base de platine supporté tel que la distribution du platine soit limitée à la surface externe du support sur une épaisseur maximale de 400 µm. L'avantage d'un tel choix réside dans le fait qu'une distribution en périphérie du support permet de limiter les réactions parasites et par conséquent d'améliorer les performances du catalyseur. Cependant, ce type de distribution ne permet que rarement l'obtention des rapports atomiques platine/modificateurs homogènes à l'échelle des particules (nanomètre). De plus, une surconcentration de phase active peut engendrer des limitations diffusionnelles au niveau du grain de catalyseur (diffusion extragranulaire) et réduire de ce fait le rendement global de la réaction.

Les brevets et publications démontrant que l'addition de promoteurs à un métal de base améliore les performances des catalyseurs sont fort nombreux. Ces éléments sont ajoutés sous différentes formes telles que sels ou composés organométalliques. On obtient généralement des catalyseurs plus actifs ou plus sélectifs, parfois plus stables que le catalyseur monométallique correspondant.

Ainsi la formulation de catalyseurs utilisés dans les procédés de transformation d'hydrocarbures, en particulier celle des catalyseurs de déshydrogénation de paraffines a fait l'objet d'un très grand nombre d'études. Parmi les promoteurs fréquemment employés, l'étain permet notamment d'accroître la sélectivité et la stabilité des catalyseurs. Ainsi, des catalyseurs à base de PtSn supportés sur alumine et utilisés en déshydrogénation de paraffines ou en réformage catalytique ont par exemple été décrits dans les brevets FR-B-2 031 984 et US-A-3 531 543.

En particulier, les catalyseurs à base de PtSn contiennent différentes formes d'étain. A l'état réduit, ces catalyseurs, supportés sur alumine, contiennent essentiellement des espèces d'étain à l'état oxydé, à savoir des espèces de l'étain divalent Sn^{II} et de l'étain tétravalent Sn^{IV} et des quantités minoritaires d'étain à l'état réduit Sn⁰ (M.C. Hobson et al, J. Catal., 142, 641-654 (1993), L.D Sharma et al, Appl. Catal. A Genneral, 168, 251-259, (1998)). Ces catalyseurs sont préparés généralement à partir d'une solution de chlorure d'étain en milieu acide (HCl, HNO3) et d'une solution d'acide hexachloroplatinique. Le rôle de l'étain présent à la surface du catalyseur à l'état d'oxydation + 2 ou plus préférentiellement + 4 est de minimiser les réactions d'isomérisation et de craquage qui se produisent au niveau des sites acides du support ainsi que de limiter la formation de coke ce qui permet d'obtenir une meilleure stabilité du catalyseur.

Une technique qui permet d'examiner la structure électronique locale de l'étain (degré d'oxydation, environnement, liaison chimique) est la spectroscopie Môssbauer qui fournit directement deux paramètres fondamentaux: "le déplacement isomérique δ" (IS) et "l'éclatement quadripolaire Δ" (QS). Le déplacement isomérique δ qui mesure la position en énergie de l'absorption Mössbauer, fonction de la densité s au noyau caractérise directement le degré d'oxydation de l'étain. L'éclatement quadripolaire Δ qui définit la forme de l'absorption, fonction de la répartition des charges environnantes caractérise le motif de coordination et donc le type de liaison chimique dans laquelle est impliqué l'étain. Chaque espèce d'étain est caractérisée par un sous spectre défini par les deux paramètres IS et QS. La spectroscopie Mössbauer donne également accès à la largeur de raie LW, par comparaison avec la largeur naturelle d'émission (0,64 mm/s) : la largeur de raie LW apporte des informations sur le degré d'ordre et sur la distribution des sites occupés par l'étain. L'intensité de l'absorption relative à chaque espèce est proportionnelle au nombre d'atomes d'étain et au facteur de Lamb Mössbauer f qui représente la probabilité d'absorption résonnante sans effet de recul ni d'élargissement thermique. Ce facteur f est donc directement relié à la rigidité du réseau et sa valeur est augmentée par un abaissement de la température de mesure. Il peut être faible à température ambiante (0,06 pour la phase métallique de β l'étain) et nécessiter donc des mesures à basse température. La proportion de chaque espèce est estimée à partir de leur contribution à l'absorption totale à condition que les fractions f d'absorption résonnante sans recul ne soient pas trop différentes.

L'ensemble des caractérisations par spectroscopie Mössbauer de catalyseurs réduits à base de PtSn supportés sur alumine ou sur silice mentionnent l'existence d'une espèce Sn⁰ contenue dans une phase de type PtₓSn_{y} (x et y variant de 1 à 4) pour laquelle l'étain est à l'état d'oxydation 0 (IS variant de 1,4 à 1,8 mm/s par rapport à BaSnO₃) sous une forme très proche des alliages massiques caractérisés par un éclatement quadripolaire faible ou nul (M.C. Hobson et al, J. Catal., 142, 641-654 (1993); Z. Huang et al, J. Catal., 159, 340-352 (1993); J.L. Margitfalvi et al, J. Catal., 190, 474-477 (2000); V.I. Kuznetov et al, J. Catal., 99, 159 (1986); R. Bacaud et al, J. Catal., 69, 399 (1981); R. Srinivasan et al, Catal. Today, 21, 83 (1994)). Sur alumine, la formation d'étain métallique, à l'état réduit, favorisée pour les plus grandes tailles de particules métalliques, supérieures à 2 nm serait responsable de la perte de performances de catalyseurs PtSn supportés sur alumine (Z. Huang et al, J. Catal., 159, 340-352, (1993), F. Yining et al, Stud. Surf. Sci. Catal., 68, 683-690, (1991)). Ainsi plusieurs documents décrivent l'utilisation de catalyseurs contenant une phase PtSn dispersée sur alumine où l'étain est essentiellement dans un état d'oxydation supérieur à celui de l'étain métallique (US 3 846 243, US 3 847 794). Dans de telles conditions, les méthodes de préparation classiquement utilisées ne permettent pas de garantir une association étroite entre l'étain et le platine, une association intime entre ces métaux dans le catalyseur à l'état réduit étant pourtant généralement recherchée pour exploiter au mieux l'effet bimétallique dans des procédés de transformation des composés organiques.

### Résumé de l'invention

L'invention concerne un procédé pour la déshydrogénation de composés organiques réalisé en présence d'un nouveau catalyseur supporté contenant au moins un métal du groupe VIII de la classification périodique des éléments et au moins de l'étain dont au moins une partie est en forte interaction avec ledit métal du groupe VIII. Le catalyseur supporté mis en oeuvre dans le procédé de l'invention est **caractérisé en ce qu**'il contient des particules métalliques, de petite taille, inférieure à 2 nm et en ce qu'au moins 10 % des espèces d'étain, présentes sur le catalyseur à l'état partiellement réoxydé, sont sous la forme d'une espèce d'étain réduite à l'état d'oxydation 0. Ladite espèce d'étain réduite est sous une forme particulière, mise en évidence par spectroscopie Mössbauer de ¹¹⁹Sn, et est caractérisée par une valeur très élevée d'éclatement quadripolaire, supérieure à 0,65 mm/s, et par un déplacement isomérique IS compris entre 0,8 et 2,6 mm/s par rapport à BaSnO₃. Cette espèce est révélée en soumettant le catalyseur réduit à une oxydation parfaitement controlée par pulses d'oxygène. Cette espèce particulière d'étain est très étroitement associée au métal du groupe VIII et est révélatrice d'une très forte interaction entre les atomes du métal du groupe VIII et au moins une fraction de l'étain dans le catalyseur à l'état réduit. Par exemple, dans le cas où le métal du groupe VIII est le platine, il se forme une phase PtₓSn_{y} où l'étain présente des valeurs de IS et de QS déterminées. De manière préférée, le catalyseur contient aussi un métal alcalin ou alcalino-terreux.

### Intérêt de l'invention

Le catalyseur mis en oeuvre dans le procédé de l'invention présente des propriétés catalytiques nettement améliorées par rapport aux catalyseurs de l'art antérieur, notamment en ce qui concerne l'activité et la stabilité. En effet, il a été découvert, de manière surprenante, que la présence en forte quantité d'une espèce d'étain réduite à l'état d'oxydation 0 et associée étroitement avec un métal du groupe VIII, dans un catalyseur bimétallique partiellement oxydé par une oxydation réalisée dans des conditions parfaitement contrôlées sous pulses d'oxygènes, est révélatrice d'une forte interaction, à l'état réduit du catalyseur, entre le métal du groupe VIII et au moins une fraction de l'étain, garantissant un effet bimétallique bénéfique sur les performances catalytiques des unités de déshydrogénation de composés organiques, en termes d'activité et de stabilité, une meilleure activité et une meilleure stabilité permettant en effet d'augmenter très sensiblement les rendements en alcènes et aromatiques, produits cibles des réactions de déshydrogénation de paraffines et de naphtènes.

### Description

Le procédé pour la déshydrogénation de composés organiques selon l'invention comprend la mise en contact d'une charge hydrocarbonée avec un catalyseur supporté comportant au moins un métal du groupe VIII de la classification périodique des éléments et de l'étain dont au moins une partie est en forte interaction avec le métal du groupe VIII dans le catalyseur à l'état réduit. On distinguera, dans la suite de la description, le catalyseur à l'état réduit du catalyseur partiellement oxydé en ce que la quantité d'étain réduit à l'état d'oxydation 0 et en association intime avec un métal du groupe VIII est plus importante dans le catalyseur partiellement oxydé.

L'invention vise la déshydrogénation de tous types de composés organiques. Plus précisément, elle vise la déshydrogénation de paraffines courtes, c'est-à-dire de composés organiques aliphatiques saturés ayant de 2 à 5 atomes de carbone, la déshydrogénation de paraffines longues, c'est-à-dire de composés organiques aliphatiques saturés ayant de 6 à 22 atomes de carbone ainsi que la déshydrogénation de naphtènes, c'est-à-dire de composés organiques cycliques saturés.

Dans le cadre de l'invention, les naphtènes sont de préférence choisis dans le groupe constitué par le méthylcyclohexane et le cyclohexane.

La charge hydrocarbonée mise en contact avec le catalyseur peut comprendre d'autres composés que ceux à déshydrogéner et en particulier elle peut contenir des aromatiques.

Le support du catalyseur utilisé pour la mise en oeuvre du procédé de l'invention comporte au moins un oxyde réfractaire qui est généralement choisi parmi les oxydes de métaux des groupes IIA, IIIA, IIIB, IVA ou IVB, de la classification périodique des éléments, tels que par exemple les oxydes de magnésium, d'aluminium, de silicium, de niobium, de titane, de zirconium et de thorium pris seuls ou en mélange entre eux ou en mélange avec des oxydes d'autres éléments de la classification périodique. Pour les réactions de déshydrogénation de composés organiques, le support préféré est l'alumine, dont la surface spécifique est avantageusement comprise entre 5 et 400 m² par gramme, et de préférence entre 50 et 350 m² par gramme. On peut aussi utiliser comme support du catalyseur utilisé dans le procédé de l'invention des zéolithes ou tamis moléculaires de type X, Y, mordénite, faujasite, ZSM-5, ZSM-4, ZSM-8, MFI, EUO, mazzite ainsi que les mélanges d'oxydes de métaux des groupes IIA, IIIA, IIIB, IVA et IVB avec du matériau zéolithique et en particulier les mélanges d'oxydes alumine-zéolithes
Le métal du goupe VIII est le métal de base catalytiquement actif du catalyseur utilisé pour la mise en oeuvre du procédé de l'invention. Préférentiellement, il s'agit d'un métal noble de la famille du platine (Pt, Pd, Rh, Ir). De manière très préférée, le métal noble est le platine. Avantageusement le catalyseur contient un premier métal noble (tel que Pt) auquel est additionné de l'iridium. Dans les réactions de déshydrogénation de composés organiques et en particulier de paraffines, le platine et l'iridium sont les métaux préférés. Le pourcentage pondéral est alors choisi entre 0,01 et 10 % et de préférence entre 0,05 et 5 %.
L'étain joue le rôle de promoteur. Le pourcentage pondéral de l'étain, compris dans le catalyseur à l'état réduit, toutes espèces comprises (réduite et oxydées), par rapport au poids total de catalyseur est choisi par exemple entre 0,01 et 2 %. Le catalyseur utilisé dans le procédé de l'invention contient très avantageusement au moins 0,1 % poids d'étain. Selon l'invention, l'étain est présent dans le catalyseur réduit essentiellement à l'état oxydé (Sn^{II} et Sn^{IV}). Une caractéristique essentielle du procédé de l'invention est l'utilisation d'un catalyseur ayant une forte proportion en étain métallique Sn⁰ par rapport aux espèces oxydées de l'étain, lorsque le catalyseur est dans un état partiellement oxydé c'est-à-dire quand le catalyseur réduit a été soumis à une oxydation parfaitement contrôlée par pulses d'oxygène. Cette espèce d'étain métallique Sn⁰ est sous une forme très particulière d'alliage métal VIII-Sn, dans lequel ledit métal du groupe VIII et l'étain sont intimement associés et sont en forte interaction avec l'oxygène. Cette espèce présente des valeurs très élevées de QS comprises entre 0,65 et 2,00 mm/s et est révélée lorsque le catalyseur est partiellement oxydé. Ainsi le catalyseur, mis en contact avec une charge hydrocarbonée, se **caractérise en ce qu'**à l'état partiellement oxydé au moins 10 % de l'étain par rapport à l'étain introduit est sous la forme d'une espèce réduite à l'état d'oxydation 0, c'est-à-dire que cette espèce réduite à l'état d'oxydation 0 représente au moins 10 % de l'étain présent dans la masse catalytique. Avantageusement, ladite espèce d'étain réduite à l'état d'oxydation 0 représente au moins 12 % de l'étain présent dans la masse catalytique. De manière préférée, elle en représente au moins 15 %, de manière très préférée, elle en représente au moins 20 % et de manière encore plus préférée, elle en représente au moins 25 %. Très préférentiellement, elle en représente au moins 30 %.
A l'état partiellement oxydé du catalyseur, ladite espèce d'étain réduite ne représente généralement pas plus de 90% de la masse catalytique. De préférence, elle n'en représente pas plus de 70% et de manière très préférée, elle n'en représente pas plus de 60%.

Par "catalyseur à l'état partiellement oxydé", on entend un catalyseur ayant été oxydé de manière parfaitement contrôlée par pulses d'oxygène. Conformément à l'invention, l'oxydation parfaitement contrôlée du catalyseur à l'état réduit permet de révéler par spectroscopie Môssbauer la présence d'une forte quantité d'étain métallique en association intime avec le métal du groupe VIII, notamment avec le platine, et l'oxygène, la présence de cette espèce d'étain réduite traduisant l'existance d'une forte interaction entre le métal du groupe VIII, de préférence le platine, et au moins une fraction de l'étain dans le catalyseur à l'état réduit. Le catalyseur de l'invention à l'état partiellement oxydé contient des espèces Sn²⁺ et ladite espèce d'étain réduite à l'état d'oxydation 0 (Sn⁰) est sous une forme particulière d'alliage métal VIII-Sn, préférentiellement sous la forme particulière d'alliage Pt-Sn.

Dans le cas où l'oxydation du catalyseur réduit est réalisée dans des conditions non contrôlées telles que par exemple une réoxydation sous air à forte pression partielle d'oxygène, la teneur en l'espèce d'étain métallique Sn⁰ sous la forme de l'alliage métal VIII-Sn, de préférence sous la forme de l'alliage PtSn, diminue de manière très sensible au profit de la formation d'espèces Sn⁴⁺. Le catalyseur est alors fortement oxydé et comprend essentiellement des espèces Sn⁴⁺. Il ne contient plus d'espèces Sn²⁺. Cette formation d'espèces Sn⁴⁺ pertube la quantification d'espèces Sn en forte interaction avec le métal du groupe VIII, de préférence avec le platine, présente dès l'état réduit, ne permettant donc pas d'apprécier les performances du catalyseur à l'état réduit.
L'oxydation contrôlée par pulse permet au contraire la formation sélective de ladite espèce d'étain réduite Sn⁰ ayant les caractéristiques Môssbauer décrites ci-dessus en évitant la formation d'espèces Sn⁴⁺.

Le catalyseur contient éventuellement également de 0,1 à 3 % poids d'au moins un métal alcalin
ou alcalino-terreux. La déshydrogénation de paraffines est très préférentiellement réalisé avec un catalyseur comprenant un tel élément (alcalin ou alcalino-terreux). Pour la déshydrogénation de paraffines courtes, on utilise préférentiellement le potassium et pour la déshydrogénation de paraffines longues, on utilise préférentiellement le lithium.
Le catalyseur peut contenir en outre au moins un élément du groupe IIIA. Le métal du groupe IIIA est choisi parmi l'indium, le gallium et le thallium, de préférence l'indium à une teneur comprise entre 0,005 et 3 % par rapport au poids total du catalyseur et plus préférentiellement entre 0,1 et 1 %. Le catalyseur utilisé pour la déshydrogénation de paraffines longues contient très avantageusement de l'indium. Le catalyseur peut en outre contenir éventuellement, par exemple, au moins un halogène ou un composé halogéné dans des proportions de l'ordre de 0,1 à 3 % poids du catalyseur. Il peut aussi éventuellement contenir au moins un métalloïde tel que le soufre dans des proportions de l'ordre de 0,01 à 2 % poids du catalyseur. Il peut aussi contenir au moins un autre élément chimique, par exemple le rhénium ou le niobium, dans des proportions de l'ordre de 0,01 à 3 % poids du catalyseur, ledit élément pouvant être introduit dans le catalyseur par toute méthode et sous toutes les formes connues de l'homme du métier.
Le catalyseur peut se présenter sous forme de billes, extrudés, trilobes ou toute forme communément utilisée. Le catalyseur utilisé dans le procédé de l'invention contient des particules métalliques de petites tailles, c'est-à-dire d'une taille inférieure à 2 nm. Ces particules métalliques contiennent le métal noble et l'étain, l'étain se trouvant majoritairement à l'état d'oxydation 0 dans des proportions telles que décrites précédemment.
Les analyses permettant d'examiner la structure électronique locale de l'étain sont conduites sur un spectromètre Mössbauer classique équipé avec une source de rayons γ Ba^{119m}SnO₃ d'activité nominale 10 mCi. Le spectromètre opère en transmission avec un transducteur de vitesse à accélération constante fonctionnant en mode triangulaire en relation avec un analyseur multicanal à 512 canaux, contrôlé par un micro-ordinateur. Le détecteur est un scintillateur à cristal NaI(T1) d'épaisseur 0,1 mm. L'échelle des vitesses est calibrée en utilisant le spectre standard à 6 raies de α-Fe obtenu avec une source ⁵⁷Co(Rh). Tous les déplacements isomériques IS sont donnés par rapport au standard BaSnO₃. Les spectres expérimentaux sont décomposés en profils Lorentzien et les différents paramètres affinés par moindres carrés à l'aide du logiciel ISO (W. Künding, Nucl. Instrum. Methods, 75, 336 (1969)).
Pour certaines analyses effectuées à basse température, il est avantageux d'utiliser un cryostat à circulation et à température variable (4 à 300 K). De telles mesures sont nécessaires pour caractériser les valeurs de f relatives à une espèce donnée.
Les analyses sont effectuées sur des catalyseurs en poudre, préalablement réduits, sous un débit d'hydrogène entre 450 et 550°C. Après retour à la température ambiante sous hydrogène et balayage sous gaz neutre comme l'hélium, le catalyseur réduit est soumis au nombre de pulses d'oxygène nécessaire pour le saturer. Les injections par pulses sont poursuivies jusqu'à l'obtention d'au moins 10 pics de surface constante (analyse chromatographique), puis on soumet le catalyseur à un balayage sous gaz neutre comme l'hélium, la cellule de traitement est scellée directement sans aucune remise à l'air. La quantité de catalyseur nécessaire tenant compte de la teneur en étain est d'au moins 2 g. Cette cellule peut être utilisée indifféremment à température ambiante ou à basse température. Le catalyseur testé en spectroscopie Mössbauer est à l'état partiellement réoxydé.

Le catalyseur utilisé dans le procédé de l'invention, et analysé par spectroscopie Mössbauer à l'état partiellement oxydé, contient de l'étain sous forme oxydée (étain divalent et tétravalent) et sous forme réduite. Selon l'invention, les espèces Sn^{IV} sont caractérisées par une valeur de déplacement isomérique IS comprise entre 0 et 0,25 mm/s et une valeur de l'éclatement quadripolaire QS comprise entre 0 et 0,80 mm/s. Les espèces Sn^{II} sont caractérisées par un IS compris entre 2,70 et 3,40 mm/s et un QS compris entre 1,60 et 2,60 mm/s. Les espèces Sn⁰ sont caractérisées par un IS compris entre 0,80 et 2,60 mm/s et un QS compris entre 0,65 et 2,00 mm/s.
Selon l'invention ladite espèce d'étain réduite à l'état d'oxydation 0 (Sn⁰) est une forme particulière d'alliage métal VIII-Sn, notamment une forme particulière d'alliage PtSn, avec des valeurs de IS, comprises entre 0,80 et 2,60 mm/s, de préférence entre 0.80 et 1.50 mm/s et de manière encore plus préférée entre 1,10 et 1,40 mm/s et avec des valeurs de QS comprises entre 0,65 et 2,00 mm/s, de préférence entre 0,80 et 2,00 mm/s, de manière préférée entre 0,90 et 1,90 mm/s et de manière encore plus préférée entre 0,95 et 1,50 mm/s. Avantageusement, ladite espèce d'étain réduite à l'état d'oxydation 0 (Sn⁰), sous forme particulière d'alliage métal VIII-Sn, et notamment sous la forme de l'alliage PtSn, et présentant les valeurs de IS et QS tels que définis précédemment n'est présente que dans le catalyseur à l'état partiellement oxydé.

Les valeurs des paramètres Mössbauer obtenues pour le catalyseur à l'état partiellement oxydé traduisent l'existence d'une forte interaction entre l'étain et le métal du groupe VIII, cette interaction étant révélée par une oxydation parfaitement contrôlée du catalyseur réduit.

L'association très étroite entre ces deux métaux dans le catalyseur utilisé pour la mise en oeuvre du procédé de l'invention permet d'exploiter au mieux les propriétés intrinsèques à chaque métal et génère ainsi un effet synergique, d'autant plus notable que l'espèce d'étain réduite à l'état d'oxydation 0 est en quantité importante et présente un QS élevé. Par exemple, lorsque le métal du groupe VIII est le platine, l'espèce d'étain réduite est contenue dans une phase particulière du type PtₓSn_{y} où ladite espèce d'étain réduite et le platine sont en association intime. Le précurseur d'étain peut être choisi, sans que cette liste soit limitative, dans le groupe des composés halogénés, des hydroxydes, des oxydes, des carbonates, des carboxylates, des nitrates et des sulfates de l'étain. Il peut être introduit sous la forme d'au moins un composé organique choisi dans le groupe formé par les complexes d'étain, et les hydrocarbylétain tels que les alkyles, les cycloalkyles, les aryles, les alkylaryles et les arylalkyles étain. Le précurseur de l'étain peut être choisi, sans que cette liste soit limitative, dans le groupe des composés halogénés, des hydroxydes, des oxydes, des carbonates, des carboxylates, des nitrates et des sulfates de composés organométalliques de l'étain. Ces composés comprennent au moins une liaison carbone-Sn. Par exemple le précurseur de l'étain peut être choisi parmi les halogénures de polyalkyles, par exemple les halogénures de triméthyle (Me₃SnX), de triéthyle (Et₃SnX), les dihalogénures de diméthyle (Me₂SnX₂), de diéthyle (Et₂SnX₂), de diisopropyle (iPr₂SnX₂), de di-n-propyle (n-Pr₂SnX₂), les trihalogénures de méthyle (MeSnX₃), d'éthyle (EtSnX₃), d'isopropyle (iPrSnX₃), de di-n-propyle (n-PrSnX₃), les hydroxydes de polyalkyles, par exemple les hydroxydes de triméthyle (Me₃SnOH), de triéthyle (Et₃SnOH) les dihydroxydes de diméthyle (Me₂Sn(OH)₂), de diéthyle (Et₂Sn(OH)₂), de disopropyle (iPr₂Sn(OH)₂), de n-propyle (n-Pr₂Sn(OH)₂), les trihydroxydes de méthyle (MeSn(OH)₃), d'éthyle (EtSn(OH)₃), de diisopropyle (iPrSn(OH)₃), les trihydroxydes de n-propyle (n-PrSn(OH)₃), les acétates de polyalkyles, par exemple les acétates de triméthyle (Me₃SnOC(O)Me), de triéthyle (Et₃SnOC(O)Me), de tributyle (Bu₃SnOC(O)Me), les oxydes de polyalkyles, par exemple les oxydes de bis triméthyle ([Me₃Sn]₂O), de bis triéthyle ([Et₃Sn]₂O), de bis tripropyle ([Pr₃Sn]₂O), de bis tributyle ([Bu₃Sn]₂O), les sulfates de polyalkyles, par exemple les sulfates de bis triméthyle ([Me₃Sn]₂SO₄), de bis diméthyle ([Me₂Sn]SO₄), les méthyl trioxo (MeSnO₃), où X représente un halogène choisi dans le groupe constitué par le fluor, le chlore, le brome et l'iode. Le précurseur de l'étain peut être choisi parmi les composés de formule générale (R1)ₓ M (R2)_{y} (R3)_{z} avec x+y+z = valence de l'étain et où R1 est choisi dans le groupe des radicaux alkyles, cycloalkyles, nitriles (CN), carbonyles (CO), aryles, alkylaryles et arylalkyles, où R2 est une fonction de la forme CₐH_{b}R'_{c}
où R' représente une fonction hydroxyde, halogénure, carboxylate, PO₃H ou SO₃H et où R3 est un groupement aquo, oxo (MO), alkoxyde (O-alkyl), hydrure, hydroxyle, alkylsulfonate, alkylsulfate, thioalkyle, N(SO₃R")₂, PR"₂ et PR"₃ où R" est un groupe alkyle. (Handbook of Physics and Chemistry, 63ème édition, 1982-83).
On entend par groupements alkyles des groupements comprenant des atomes de carbone et d'hydrogène, saturés, linéaires, ramifiés ou cycliques. On entend par groupements aryles les groupements aromatiques.
On peut remplacer dans les composés cités ci-dessus au moins un groupement alkyle par un groupement alkényle, c'est-à-dire un groupement comprenant des atomes de carbone et d'hydrogène, insaturé, linéaire, ramifié ou cyclique, par exemple un groupement allyle.

Les précurseurs préférés de l'étain sont les composés organométalliques de type SnR₄ (R = groupe alkyle) ou encore les halogénures de polyalkyles tels que Me₃SnCl, Me₂SnCl₂, MeSnCl₃, Et₃SnCl, Et₂SnCl₂, EtSnCl₃, iPrSnCl₂ et les hydroxydes Me₃SnOH, Me₂Sn(OH)₂, Et₃SnOH, Et₂Sn(OH)₂, les oxydes [Bu₃Sn]₂O, l'acétate Bu₃SnOC(O)Me. Ces halogénures de polyalkyles comportent au moins une liaison carbone-Sn et au moins une fonction hydrosoluble, ce qui les rend solubles dans des solvants aqueux, facilitant ainsi la mise en oeuvre lors de la préparation du catalyseur.
Le composé du métal du groupe VIII peut être introduit sous la forme d'un complexe minéral ou organique et choisi par exemple, dans le cas où le métal du groupe VIII est le platine, parmi l'acide hexachloroplatinique, l'acide hexahydroxyplatinique, le platine dihydroxytétramine, le diamino nitrite de platine, ou parmi des complexes organométalliques tels que le bis acétylacétonate de platine.
La préparation du catalyseur comprend l'introduction, simultanée ou successive, dans n'importe quel ordre, du métal du groupe VIII, de l'étain, éventuellement du métal alcalin ou de l'alcalino-terreux, éventuellement de l'halogène ou du composé halogéné, éventuellement du métalloïde, éventuellement d'un autre élément chimique. Lors d'une introduction successive des métaux, lorsque le premier métal est introduit, l'homme de l'art sait ensuite adapter les conditions d'introduction des autres éléments de manière à obtenir un catalyseur ayant les caractéristiques telles que définies précédemment.
L'introduction des métaux peut être réalisée lors de toutes les étapes de fabrication du catalyseur selon les techniques de l'art antérieur. Par exemple l'étain peut être ajouté à un sol d'alumine (US-A-3 929 683) ou lors de la mise en forme du support suivant par exemple, les procédures de mise en forme par extrusion (US-A-3 917 808) ou par coagulation en gouttes (US-A-3 558 508).
Selon un mode de mise en oeuvre préféré de préparation du catalyseur, le support est préférentiellement d'abord imprégné à l'aide d'une solution d'un métal alcalin ou alcalino-terreux, le mélange est filtré puis le produit récupéré est séché et calciné. Le solide obtenu est imprégné à l'aide d'une solution aqueuse saturée par du CO₂, contenant au moins un précurseur de l'étain sous forme SnCl₂ ou de préférence sous forme de composés organométalliques ayant au moins une liaison carbone-étain tels que par exemple les halogénures de polyalkyles tels que Me₃SnCl, Me₂SnCl₂, MeSnCl₃, Et₃SnCl, Et₂SnCl₂, EtSnCl₃, iPrSnCl₂ et les hydroxydes Me₃SnOH, Me₂Sn(OH)₂, Et₃SnOH, Et₂Sn(OH)₂, les oxydes [Bu₃Sn]₂O, l'acétate Bu₃SnOC(O)Me. Après avoir laissé en contact le solide et la solution d'imprégnation pendant plusieurs heures, le produit est filtré puis éventuellement soumis à une étape de séchage à 120°C et éventuellement de calcination entre 300 et 600 °C, de préférence entre 450 et 550°C. Le solide ainsi obtenu est imprégné de préférence par une solution organique d'au moins un composé de métal du groupe VIII, le volume de la solution étant en excès par rapport au volume de rétention du support. Après quelques heures de mise en contact, le produit obtenu est ensuite séché puis calciné sous air entre 300 et 600°C, de préférence en effectuant un balayage d'air durant plusieurs heures.

Selon un autre mode de mise en oeuvre préféré de préparation du catalyseur, le métal de base catalytiquement actif tel que le platine est déposé en plusieurs étapes avant le dépôt d'étain de manière à déposer sélectivement l'étain sur des particules de taille controlée, c'est-à-dire sur des particules de taille supérieure à celles du catalyseur final. Par exemple, le support est imprégné par une solution organique contenant au moins un composé organométallique de platine tel que le bis acétylacétonate de platine (Pt(acac)₂), le volume de la solution étant de préférence en excès par rapport au volume de rétention du support. Après avoir laissé en contact le solide et la solution d'imprégnation pendant plusieurs heures, le produit est filtré, puis séché et calciné sous air entre 300 et 600°C, de préférence entre 400 et 500°C, en effectuant avantageusement un balayage d'air durant plusieurs heures. Il est ensuite réduit sous un balayage sous hydrogène entre 300 et 600°C, de préférence entre 350 et 500°C. Le catalyseur est ensuite transféré sans remise à l'air dans le réacteur d'imprégnation, pour déposer à nouveau du platine suivant exactement la même procédure que précedemment. Ceci peut être pratiqué plusieurs fois. Pour le dépôt d'étain, le solide obtenu est ensuite transféré sans remise à l'air, dans un réacteur où l'imprégnation d'étain se fait par mise en contact pendant plusieurs heures d'une solution aqueuse ou organique d'un composé organométallique de l'étain, le volume de la solution étant de préférence en excès par rapport au volume de rétention du support. La réaction est avantageusement effectuée en assurant un balayage d'hydrogène dans la solution d'imprégnation. Le solide ainsi obtenu, est filtré, séché puis réduit sous courant d'hydrogène entre 300 et 600°C. Si le catalyseur contient un métal alcalin ou alcalino-terreux, celui-ci peut être ajouté à n'importe quel moment de la préparation. De préférence, il est introduit une fois que l'imprégnation de l'étain a eu lieu. Le catalyseur est finalement calciné.
Pour la déshydrogénation de paraffines courtes, le catalyseur, quel que soit la manière dont il a été préparé, est soumis à une étape finale consistant en un traitement d'oxychloration. Le traitement d'oxychloration est généralement réalisé sous un débit de gaz comprenant de l'oxygène, du chlore et éventuellement de l'eau selon toute technique connue de l'homme du métier (US-A- 3 875 049).
Au contraire, pour des procédés de déshydrogénation de paraffines longues, il n'est pas conseillé de réaliser un traitement d'oxychloration du catalyseur, car la présence de chlore diminue les performances de tels procédés. Il est par ailleurs préférable d'utiliser des précurseurs des métaux compris dans le catalyseur contenant peu voir pas de chlore. Par exemple, comme précurseur du platine, on utilise du bis acéthylacétonate de platine et comme précurseur de l'étain on utilise Me₃SnCl ou Me₃SnOH.
Avant utilisation et quel que soit le type de composés à déshydrogéner, on réduit le catalyseur sous hydrogène par exemple entre 200 et 600°C afin d'obtenir une phase métallique active. La procédure de ce traitement consiste par exemple en une montée lente de la température sous courant d'hydrogène jusqu'à la température maximale de réduction, comprise par exemple entre 200 et 600°C, de manière préférée entre 250 et 550°C et de manière plus préférée entre 350 et 550°C, suivie d'un maintien par exemple durant 1 à 6 heures à cette température.
Cette réduction peut être effectuée aussitôt après une calcination ou ultérieurement chez l'utilisateur. Il est aussi possible de réduire directement le produit séché chez l'utilisateur. Toute autre méthode de préparation conduisant à un catalyseur réduit présentant une forte interaction entre au moins une fraction de l'étain et un métal du groupe VIII et contenant à l'état partiellement oxydé au moins 10 % de l'étain sous la forme d'une espèce d'étain réduite à l'état d'oxydation 0 où l'espèce réduite d'étain Sn⁰ présente un déplacement isomérique compris entre 0,80 et 2,60 mm/s et un éclatement quadripolaire compris entre 0,65 et 2,00 mm/s convient.
Dans le cas où le catalyseur de la présente invention contient du soufre, le soufre est introduit sur le catalyseur mis en forme, calciné, contenant le ou les métaux cités précédemment, soit *in-situ* avant la réaction catalytique, soit *ex-situ*. La sulfuration éventuelle intervient après la réduction. Dans le cas d'une sulfuration *in-situ*, la réduction, si le catalyseur n'a pas été préalablement réduit, intervient avant la sulfuration. Dans le cas d'une sulfuration *ex-situ*, on effectue la réduction puis la sulfuration. La sulfuration s'effectue en présence d'hydrogène en utilisant tout agent sulfurant bien connu de l'homme de métier, tel que par exemple le sulfure de diméthyle ou le sulfure d'hydrogène. Par exemple, le catalyseur est traité avec une charge contenant du sulfure de diméthyle en présence d'hydrogène, avec une concentration telle que le rapport atomique soufre/métal soit de 1,5. Le catalyseur est ensuite maintenu pendant environ 3 heures à environ 400 °C sous débit d'hydrogène avant l'injection de la charge.

Les différents procédés de déshydrogénation de paraffines et de naphtènes se différencient par le choix des conditions opératoires et de la composition de la charge. L'ajustement des conditions opératoires, en fonction de la nature de la charge à traiter, se fait de façon à obtenir la meilleure adéquation pression-température-rendement et activité de façon connue de l'homme du métier.
La réaction de déshydrogénation des paraffines s'effectue en général à une pression comprise entre 0.02 et 2 MPa, de préférence comprise entre 0,1 et 1 MPa et à une température comprise entre 400 et 800°C en fonction de la nature de la charge. La température est avantageusement comprise entre 400 et 550°C pour une charge comprenant essentiellement de l'isopentane. La température est avantageusement comprise entre 450 et 550 °C pour une charge comprenant principalement des paraffines comportant de 9 à 22 atomes de carbone par molécule. La charge peut aussi contenir des hydrocarbures insaturés comportant de 3 à 22 atomes de carbone par molécule. Le débit massique de charge traitée par unité de masse de catalyseur est généralement compris entre 0,5 et 100 kg/kg/h. Il peut être avantageux d'utiliser l'hydrogène comme diluant. Le rapport molaire hydrogène/hydrocarbure est généralement compris entre 0 et 20, de préférence entre 0 et 6.
La réaction de déshydrogénation des naphtènes s'effectue en général à une pression comprise entre 0,1 et 2 MPa, de préférence comprise entre 0,1 et 1 MPa et à une température comprise entre 200 et 400 °C en fonction de la nature de la charge. Le débit massique de charge traitée par unité de masse de catalyseur est généralement compris entre 0,5 et 100 kg/kg/h.
Les exemples qui suivent illustrent l'invention sans toutefois en limiter la portée.

### EXEMPLE 1 (non conforme à l'invention)

On prépare un catalyseur A renfermant 0,35 % poids de platine, 0,55 % poids d'étain, 1 % poids de chlore et 1% poids de potassium déposé sur un support d'alumine gamma dont la surface spécifique est de 200 m²/g.

A 100 g de support alumine on ajoute 500 cm³ d'une solution aqueuse contenant du chlorure d'étain, en présence d'acide chlorhydrique et d'acide nitrique. On laisse en contact 3 heures, on filtre, on séche à 120°C puis on calcine pendant 2 heures à 500°C sous un débit d'air de 100 litres par heure. On met ensuite le solide en contact avec 500 cm³ d'une solution aqueuse d'acide hexachloroplatinique et d'acide chlorhydrique. On laisse en contact 3 heures puis on essore. On sèche 1 heure à 120°C puis on calcine pendant 2 heures à 500 °C sous un débit d'air de 100 litres par heure. On ajoute ensuite 100 cm³ d'une solution aqueuse de carbonate de potassium puis on sèche 1 h à 120°C puis on calcine pendant 2 heures à 500 °C sous un débit d'air de 100 litres par heure.
Le catalyseur A est ensuite réduit à 500 °C pendant 4 heures sous un débit d'hydrogène de 100 litres par heure. L'analyse par microscopie électronique à balayage (MEB) montre la très bonne dispersion de la phase métallique avec des tailles de particules inférieures à 1,2 nm.

### EXEMPLE 2 (selon l'invention)

On prépare un catalyseur B de même formulation que le catalyseur A.
Le catalyseur B est préparé par dépôt de platine en deux étapes avant l'étain. On met en contact 100 g de support alumine avec 500 cm³ d'une solution organique de bis acétylacétonate de platine. On laisse en contact 12 heures puis on essore. On séche 1 h à 120°C puis on calcine pendant 2 heures à 350°C sous un débit d'air de 100 litres par heure. On réduit ensuite le catalyseur à 450°C pendant 4 heures sous un débit d'hydrogène de 100 litres par heure. Après cette étape de réduction, le solide est transféré sans remise à l'air dans un réacteur contenant 500 cm³ d'une solution organique de bis acétylacétonate de platine. On laisse en contact 12 heures puis on essore. On séche 1 h à 120°C puis on calcine pendant 2 heures à 350°C sous un débit d'air de 100 litres par heure. On réduit ensuite le catalyseur à 450°C pendant 4 heures sous un débit d'hydrogène de 100 litres par heure. Le solide ainsi obtenu est ensuite transféré sans remise à l'air dans un réacteur contenant 500 cm³ d'une solution organique contenant une quantité nécessaire de tétrabutylétain pour déposer 0.55 % poids d'étain sur le catalyseur en présence d'un bullage d'hydrogène à 20°C. Après 24 heures de mise en contact, le mélange réactionnel est filtré, lavé puis séché à 70°C. Le catalyseur est ensuite réduit pendant 4 heures à 450°C sous 100 litres par heure d'hydrogène. On ajoute ensuite 100 cm³ d'une solution aqueuse de carbonate de potassium puis on séche 1 h à 120°C puis on calcine pendant 2 heures à 500°C sous un débit d'air de 100 litres par heure. Le catalyseur est ensuite soumis à un traitement d'oxychloration à 500°C pendant 4 heures sous un débit de 100 litres par heure d'air contenant la quantité de chlore nécessaire pour injecter en tout 1 % poids de chlore et une quantité d'eau corespondant au rapport molaire H2O/Cl de 20.
Le catalyseur B est ensuite réduit à 500 °C pendant 4 heures sous un débit d'hydrogène de 100 litres par heure. L'analyse par microscopie électronique à balayage (MEB) montre la très bonne dispersion de la phase métallique avec des tailles de particules inférieures à 1,2 nm.

### EXEMPLE 3

Pour la caractérisation par spectrométrie Mössbauer de ¹¹⁹Sn, on traite les catalyseurs A et B réduits, obtenus précédemment, à l'aide d'un appareillage χsorb qui permet de pratiquer des mesures de chimisorption d'oxygène en dynamique en liaison avec une analyse chromatographique en ligne fournissant la consommation d'oxygène. Le volume de la cellule utilisée est d'environ 10 cm³.

Dans chaque cas, on active 2 grammes de catalyseur à 500 °C pendant 4 heures sous débit d'hydrogène. Après retour à la température ambiante sous hydrogène et balayage sous débit l'hélium, le catalyseur est soumis au nombre de pulses d'oxygène nécessaire pour saturer le catalyseur c'est-à-dire jusqu'à ce qu'apparaissent des pics de surface constante indiquant la consommation totale d'oxygène. Le volume d'un pulse d'oxygène pur est de 0.22 cm³. Les injections par pulses sont poursuivies jusqu'à l'obtention d'au moins 10 pics de surface constantes, correspondant à l'oxygène qui n'a pas réagit avec le catalyseur. Ensuite après balayage sous l'hélium à température ambiante, la cellule de traitement est scellée directement sans aucune remise à l'air. Les analyses par spectrométrie Môssbauer sont réalisées avec cette cellule qui est ensuite refroidie à la température de l'azote liquide dans le cryostat à circulation.
L'enregistrement du spectre est alors réalisé en transmission selon le dispositif décrit précédemment. Le temps d'acquisition des données est choisi pour avoir le meilleur rapport signal / bruit. Dans les exemples présentés il est de 48 heures.

Les résultats comportant l'identification, les caractéristiques et les teneurs des différentes espèces d'étain présentes pour les catalyseurs A et B réduits puis partiellement réoxydés de manière contrôlée par pulses d'oxygène sont rassemblés dans le tableau 1:
Les résultats comportant l'identification, les caractéristiques et les teneurs des différentes espèces d'étain présentes dans les catalyseurs A et B réduits sont rassemblés dans le tableau 1:

**Tableau 1**

| Catalyseur | attribution | IS | QS | LW | Proportion |
|---|---|---|---|---|---|
| | espèces | (mm/s) | (mm/s) | (mm/s) | (% molaire) |
| A | Sn^{IV} | -0,03 (0,01) | 0,55 (0,01) | 0,82 (0,02) | 63,4 |
| | Sn^{II} | 3,33 (0,02) | 2,11 (0,08) | 0,82 (0,04) | 28,5 |
| | Sn⁰ | 1,55 (0,08) | 1,12 (0,01) | 1,07 (0,09) | 8,1 |
| | | | | | |
| B | Sn^{IV} | 0,03 (0,01) | 0,48 (0,01) | 0,86 (0,01) | 59,9 |
| | Sn^{II} | 3,17 (0,02) | 1,15 (0,08) | 0,86 (0,01) | 4,2 |
| | Sn⁰ | 1,29 (0,02) | 1,19 (0,03) | 1,08 (0,04) | 35,9 |

| | | | | | |
|---|---|---|---|---|---|
| 1S : déplacement isomérique δ par rapport à BaSnO₃. QS: éclatement quadripolaire Δ LW: largeur des raies à mi-hauteur Les valeurs entre parenthèses correspondent aux écarts types. | | | | | |

Comme on peut le constaster, pour une même formulation, le catalyseur B selon l'invention, contient une teneur de l'espèce Sn⁰ contenue dans la phase PtₓSny (QS de 1.19 mm/s) très supérieure à celle déterminée pour le catalyseur A non conforme à l'invention et qui correspond aux catalyseurs de l'art antérieur.

### EXEMPLE 4

Les catalyseurs A et B, à l'état réduit, décrits précédemment sont soumis à un test de déshydrogénation d'isobutane dans un réacteur tubulaire isotherme en quartz. 1 g de catalyseur est réduit à 550°C durant 2 heures sous un débit d'hydrogène de 2 litres par heure. Après injection de la charge on stabilise la température à 550°C. L'analyse des effluents gazeux se fait en ligne par chromatographie en phase gazeuse. Les conditions opératoires sont les suivantes:

| | |
|---|---|
| charge | iC4 Air Liquide N3 5 |
| température | 550°C |
| pression totale | 0.1 MPa |
| débit de charge | 200 g par g de catalyseur |
| Hydrogène/charge | 1 (molaire) |

Les performances obtenues sont reportées dans le tableau 2:

**Tableau 2**

| Catalyseur | Durée | Conversion | Sélectivité | Rendement |
|---|---|---|---|---|
| | (heure) | (% poids) | (% poids) | (% poids) |
| | | Isobutane | isobutène | isobutène |
| A | 1 | 25,2 | 95,5 | 24,1 |
| | 4 | 19,5 | 96,1 | 18,7 |
| | 8 | 13,8 | 96,3 | 13,3 |
| | | | | |
| B | 1 | 25,8 | 95,6 | 24,7 |
| | 4 | 23,9 | 96,0 | 22,9 |
| | 8 | 16,9 | 96,1 | 16,2 |

Les rendements en isobutène produits par le catalyseur B pour différentes durées de réactions sont nettement supérieurs à ceux du catalyseur A. La conversion élevée de l'isobutane avec le catalyseur B témoigne de la meilleure activité et de la meilleure stabilité du catalyseur B par rapport au catalyseur A.

## Revendications

1. Procédé pour la déshydrogénation de composés organiques comprenant la mise en contact d'une charge hydrocarbonée avec un catalyseur, à l'état réduit, contenant au moins un support, au moins un métal du groupe VIII, et au moins de l'étain dont une partie au moins est sous forme d'une espèce réduite à l'état d'oxydation 0 lorsque ledit catalyseur est à l'état partiellement oxydé, ladite espèce présentant un déplacement isomérique IS compris entre 0,80 et 2,60 mm/s et un éclatement quadripolaire compris entre 0,65 et 2,00 mm/s, ledit procédé étant **caractérisé en ce qu'**à l'état partiellement oxydé du catalyseur ladite espèce d'étain réduite à l'état d'oxydation 0 représente au moins 10 % de l'étain présent dans la masse catalytique.

2. Procédé selon la revendication 1 **caractérisé en ce qu'**à l'état partiellement oxydé ladite espèce d'étain réduite à l'état d'oxydation 0 représente au moins 12 % de l'étain présent.

3. Procédé selon l'une des revendications 1 ou 2 **caractérisé en ce qu'**à l'état partiellement oxydé ladite espèce d'étain réduite à l'état d'oxydation 0 représente au moins 15 % de l'étain présent.

4. Procédé selon l'une des revendications 1 à 3 **caractérisé en ce qu'**à l'état partiellement oxydé ladite espèce d'étain réduite à l'état d'oxydation 0 représente au moins 20 % de l'étain présent.

5. Procédé selon l'une des revendications 1 à 4 **caractérisé en ce qu'**à l'état partiellement oxydé ladite espèce d'étain réduite à l'état d'oxydation 0 représente au moins 25 % de l'étain présent.

6. Procédé selon l'une des revendications 1 à 5 **caractérisé en ce qu'**à l'état partiellement oxydé ladite espèce d'étain réduite à l'état d'oxydation 0 représente au moins 30 % de l'étain présent.

7. Procédé selon l'une des revendications 1 à 6 **caractérisé en ce que** ladite espèce d'étain réduite à l'état d'oxydation 0 présente un déplacement isomérique compris entre 0,80 et 1,50 mm/s.

8. Procédé selon l'une des revendications 1 à 7 **caractérisé en ce que** ladite espèce d'étain réduite à l'état d'oxydation 0 présente un éclatement quadripolaire compris entre 0,80 et 2,00 mm/s.

9. Procédé selon l'une des revendications 1 à 8 **caractérisé en ce que** ladite espèce d'étain réduite à l'état d'oxydation 0 présente un éclatement quadripolaire compris entre 0,90 et 1,90 mm/s.

10. Procédé selon l'une des revendications 1 à 9 **caractérisé en ce que** ladite espèce d'étain réduite à l'état d'oxydation 0 présente un éclatement quadripolaire compris entre 0,95 et 1,50 mm/s.

11. Procédé selon l'une des revendications 1 à 10 **caractérisé en ce qu'**à l'état partiellement oxydé le catalyseur contient des espèces Sn²⁺.

12. Procédé selon l'une des revendications 1 à 11 **caractérisé en ce que** le catalyseur comprend au moins un métal alcalin ou alcalino-terreux.

13. Procédé selon l'une des revendications 1 à 12 **caractérisé en ce que** le catalyseur comprend au moins un halogène ou un composé halogéné.

14. Procédé selon l'une des revendications 1 à 13 **caractérisé en ce que** le catalyseur comprend au moins un métalloïde.

15. Procédé selon l'une des revendications 1 à 14 **caractérisé en ce que** le métal du groupe VIII présent dans le catalyseur est le platine.

16. Procédé selon l'une des revendications 1 à 15 **caractérisé en ce que** le support du catalyseur est l'alumine.

17. Procédé selon l'une des revendications 1 à 16 **caractérisé en ce que** le catalyseur contient des particules métalliques de taille inférieure à 2 nm.

18. Procédé selon l'une des revendications 1 à 17 **caractérisé en ce que** les composés organiques sont des paraffines courtes ayant de 2 à 5 atomes de carbone.

19. Procédé selon selon l'une des revendications 1 à 17 **caractérisé en ce que** les composés organiques sont des paraffines longues ayant de 6 à 22 atomes de carbone.

20. Procédé selon l'une des revendications 18 à 19 **caractérisé en ce que** la charge hydrocarbonée à traiter est mise en contact avec le catalyseur sous une pression comprise entre 0,02 et 2 MPa, à une température comprise entre 400 et 800°C et avec un débit massique de charge traitée par unité de catalyseur compris entre 0,5 et 100 kg/kg/heure.

21. Procédé selon l'une des revendications 1 à 17 **caractérisé en ce que** les composés organiques sont des naphtènes.

22. Procédé selon la revendication 21 **caractérisé en ce que** les naphtènes sont choisis dans le groupe constitué par le méthylcyclohexane et le cyclohexane.

23. Procédé selon l'une des revendications 21 à 22 **caractérisé en ce que** la charge hydrocarbonée à traiter est mise en contact avec le catalyseur sous une pression comprise entre 0,1 et 2 MPa, à une température comprise entre 200 et 400°C et avec un débit massique de charge traitée par unité de catalyseur compris entre 0,5 et 100 kg/kg/heure.

## Claims

1. A process for dehydrogenating organic compounds, comprising bringing a hydrocarbon feed into contact with a catalyst in the reduced state, comprising at least one support, at least one group VIII metal, and at least tin, at least a portion of which is in the form of a reduced species with oxidation state 0 when said catalyst is in the partially oxidised state, said species having an isomer shift IS in the range 0.80 to 2.60 mm/s and a quadrupolar splitting in the range 0.65 to 2.00 mm/s, said process being **characterized in that** in the partially oxidised state of the catalyst, said reduced tin species with oxidation state 0 represents at least 10% of the tin present in the catalytic mass.

2. A process according to claim 1, **characterized in that** in the partially oxidised state, said reduced tin species with oxidation state 0 represents at least 12% of the tin present.

3. A process according to claim 1 or claim 2, **characterized in that** in the partially oxidised state, said reduced tin species with oxidation state 0 represents at least 15% of the tin present.

4. A process according to any one of claims 1 to 3, **characterized in that** in the partially oxidised state, said reduced tin species with oxidation state 0 represents at least 20% of the tin present.

5. A process according to any one of claims 1 to 4, **characterized in that** in the partially oxidised state, said reduced tin species with oxidation state 0 represents at least 25% of the tin present.

6. A process according to any one of claims 1 to 5, **characterized in that** in the partially oxidised state, said reduced tin species with oxidation state 0 represents at least 30% of the tin present.

7. A process according to any one of claims 1 to 6, **characterized in that** said reduced tin species with oxidation state 0 has an isomer shift in the range 0.80 to 1.50 mm/s.

8. A process according to any one of claims 1 to 7, **characterized in that** said reduced tin species with oxidation state 0 has a quadrupolar splitting in the range 0.80 to 2.00 mm/s.

9. A process according to any one of claims 1 to 8, **characterized in that** said reduced tin species with oxidation state 0 has a quadrupolar splitting in the range 0.90 to 1.90 mm/s.

10. A process according to any one of claims 1 to 9, **characterized in that** said reduced tin species with oxidation state 0 has a quadrupolar splitting in the range 0.95 to 1.50 mm/s.

11. A process according to any one of claims 1 to 10, **characterized in that** in the partially oxidised state, the catalyst contains Sn²⁺ species.

12. A process according to any one of claims 1 to 11, **characterized in that** the catalyst comprises at least one alkali metal or alkaline-earth metal.

13. A process according to any one of claims 1 to 12, **characterized in that** the catalyst comprises at least one halogen or halogenated compound.

14. A process according to any one of claims 1 to 13, **characterized in that** the catalyst comprises at least one metalloid.

15. A process according to any one of claims 1 to 14, **characterized in that** the group VIII metal of the catalyst is platinum.

16. A process according to any one of claims 1 to 15, **characterized in that** the catalyst support is alumina.

17. A process according to any one of claims 1 to 16, **characterized in that** the catalyst contains metallic particles less than 2 nm in size.

18. A process according to any one of claims 1 to 17, **characterized in that** the organic compounds are short chain paraffins containing 2 to 5 carbon atoms.

19. A process according to any one of claims 1 to 17, **characterized in that** the organic compounds are long chain paraffins containing 6 to 22 carbon atoms.

20. A process according to claim 18 or claim 19, **characterized in that** the hydrocarbon feed to be treated is brought into contact with the catalyst at a pressure in the range 0.02 to 2 MPa, at a temperature in the range 400°C to 800°C and at a mass flow rate of treated feed per unit mass of catalyst in the range 0.5 to 100 kg/kg/hour.

21. A process according to any one of claims 1 to 17, **characterized in that** the organic compounds are naphthenes.

22. A process according to claim 21, **characterized in that** the naphthenes are selected from the group formed by methylcyclohexane and cyclohexane.

23. A process according to claim 21 or claim 22, **characterized in that** the hydrocarbon feed to be treated is brought into contact with the catalyst at a pressure in the range 0.1 to 2 MPa, at a temperature in the range 200°C to 400°C and at a mass flow rate of treated feed per unit mass of catalyst in the range 0.5 to 100 kg/kg/hour.

## Patentansprüche

1. Verfahren zur Dehydrierung organischer Verbindungen, das das Inkontaktbringen einer Kohlenwasserstoffbeschickung mit einem Katalysator in reduziertem Zustand umfasst, der mindestens einen Träger, mindestens ein Metall der Gruppe VIII und mindestens Zinn enthält, wovon mindestens ein Teil in Form einer reduzierten Spezies im Oxidationszustand 0 ist, wenn der Katalysator in teilweise oxidiertem Zustand ist, wobei die Spezies eine Isomerieverschiebung IS im Bereich zwischen 0,80 und 2,60 mm/s und eine Quadrupolaufspaltung im Bereich zwischen 0,65 und 2,00 mm/s aufweist, wobei das Verfahren **dadurch gekennzeichnet ist, dass** im teilweise oxidierten Zustand des Katalysators die reduzierte Zinnspezies im Oxidationszustand 0 mindestens 10 % des Zinns darstellt, das in der katalytischen Masse vorhanden ist.

2. Verfahren nach Anspruch 1, **dadurch gekennzeichnet, dass** im teilweise oxidierten Zustand die reduzierte Zinnspezies im Oxidationszustand 0 mindestens 12 % des vorhandenen Zinns darstellt.

3. Verfahren nach einem der Ansprüche 1 oder 2, **dadurch gekennzeichnet, dass** im teilweise oxidierten Zustand die reduzierte Zinnspezies im Oxidationszustand 0 mindestens 15 % des vorhandenen Zinns darstellt.

4. Verfahren nach einem der Ansprüche 1 bis 3, **dadurch gekennzeichnet, dass** im teilweise oxidierten Zustand die reduzierte Zinnspezies im Oxidationszustand 0 mindestens 20 % des vorhandenen Zinns darstellt.

5. Verfahren nach einem der Ansprüche 1 bis 4, **dadurch gekennzeichnet, dass** im teilweise oxidierten Zustand die reduzierte Zinnspezies im Oxidationszustand 0 mindestens 25 % des vorhandenen Zinns darstellt.

6. Verfahren nach einem der Ansprüche 1 bis 5, **dadurch gekennzeichnet, dass** im teilweise oxidierten Zustand die reduzierte Zinnspezies im Oxidationszustand 0 mindestens 30 % des vorhandenen Zinns darstellt.

7. Verfahren nach einem der Ansprüche 1 bis 6, **dadurch gekennzeichnet, dass** die reduzierte Zinnspezies im Oxidationszustand 0 eine Isomerieverschiebung im Bereich zwischen 0,80 und 1,50 mm/s aufweist.

8. Verfahren nach einem der Ansprüche 1 bis 7, **dadurch gekennzeichnet, dass** die reduzierte Zinnspezies im Oxidationszustand 0 eine Quadrupolaufspaltung im Bereich zwischen 0,80 und 2,00 mm/s aufweist.

9. Verfahren nach einem der Ansprüche 1 bis 8, **dadurch gekennzeichnet, dass** die reduzierte Zinnspezies im Oxidationszustand 0 eine Quadrupolaufspaltung im Bereich zwischen 0,90 und 1,90 mm/s aufweist.

10. Verfahren nach einem der Ansprüche 1 bis 9, **dadurch gekennzeichnet, dass** die reduzierte Zinnspezies im Oxidationszustand 0 eine Quadrupolaufspaltung im Bereich zwischen 0,95 und 1,50 mm/s aufweist.

11. Verfahren nach einem der Ansprüche 1 bis 10, **dadurch gekennzeichnet, dass** der Katalysator im teilweise oxidierten Zustand Sn²⁺-Spezies enthält.

12. Verfahren nach einem der Ansprüche 1 bis 11, **dadurch gekennzeichnet, dass** der Katalysator mindestens ein alkalisches oder erdalkalisches Metall umfasst.

13. Verfahren nach einem der Ansprüche 1 bis 12, **dadurch gekennzeichnet, dass** der Katalysator mindestens ein Halogen oder eine halogenierte Verbindung umfasst.

14. Verfahren nach einem der Ansprüche 1 bis 13, **dadurch gekennzeichnet, dass** der Katalysator mindestens ein Metalloid umfasst.

15. Verfahren nach einem der Ansprüche 1 bis 14, **dadurch gekennzeichnet, dass** das Metall der Gruppe VIII, das in dem Katalysator vorhanden ist, Platin ist.

16. Verfahren nach einem der Ansprüche 1 bis 15, **dadurch gekennzeichnet, dass** der Träger des Katalysators Aluminiumoxid ist.

17. Verfahren nach einem der Ansprüche 1 bis 16, **dadurch gekennzeichnet, dass** der Katalysator Metallpartikel mit einer Größe von weniger als 2 nm enthält.

18. Verfahren nach einem der Ansprüche 1 bis 17, **dadurch gekennzeichnet, dass** die organischen Verbindungen kurzkettige Paraffine sind, die 2 bis 5 Kohlenstoffatome haben.

19. Verfahren nach einem der Ansprüche 1 bis 17, **dadurch gekennzeichnet, dass** die organischen Verbindungen langkettige Paraffine sind, die 6 bis 22 Kohlenstoffatome haben.

20. Verfahren nach einem der Ansprüche 18 bis 19, **dadurch gekennzeichnet, dass** die zu behandelnde Kohlenwasserstoffbeschickung mit dem Katalysator unter einem Druck im Bereich zwischen 0,02 und 2 MPa, bei einer Temperatur im Bereich zwischen 400 und 800 °C und mit einem Massenstrom der behandelten Beschickung je Katalysatoreinheit im Bereich zwischen 0,5 und 100 kg/kg/Stunde in Kontakt gebracht wird.

21. Verfahren nach einem der Ansprüche 1 bis 17, **dadurch gekennzeichnet, dass** die organischen Verbindungen Naphtene sind.

22. Verfahren nach Anspruch 21, **dadurch gekennzeichnet, dass** die Naphtene aus der Gruppe bestehend aus Methylcyclohexan und Cyclohexan ausgewählt sind.

23. Verfahren nach einem der Ansprüche 21 bis 22, **dadurch gekennzeichnet, dass** die zu behandelnde Kohlenwasserstoffbeschickung mit dem Katalysator unter einem Druck im Bereich zwischen 0,1 und 2 MPa, bei einer Temperatur im Bereich zwischen 200 und 400 °C und mit einem Massenstrom der behandelten Beschickung je Katalysatoreinheit im Bereich zwischen 0,5 und 100 kg/kg/Stunde in Kontakt gebracht wird.
